# EUROPEAN PATENT APPLICATION

(11) **EP 2 664 351 A2**
(43) Date of publication of application: **20.11.2013**
(21) Application number: 12734380.4
(22) Date of filing: 03.01.2012
(51) Int. Cl.: A61M 1/36

(54) **WARMER FOR MEDICAL TREATMENT**

(30) Priority: 11.01.2011 KR 20110002828; 22.06.2011 KR 20110060539
(71) Applicant: Park, Jae-Sang, Seoul 137-937 (KR)
(72) Inventor: Park, Jae-Sang, Seoul 137-937 (KR)
(74) Representative: Epping - Hermann - Fischer
(86) International application number: PCT/KR2012/000044
(87) International publication number: WO 2012/096468

(57) **Abstract**

The present invention relates to a warmer for medical treatment. The warmer for medical treatment includes: a heater including a resist pattern for generating heat on front and back surfaces of the heater; a partition and cover respectively disposed on the front and back surfaces of the heater to surround the heater, thereby forming a passage, through which a fluid flows, together with the heater; a first connection part for introducing the fluid into the passage; a second connection part for discharging the fluid from the passage; a pore defined in the cover; a filter disposed on an inner surface of a portion of the cover in which the pore is defined; and an air discharger coupled to the cover to discharge air contained in the fluid through the filter and the pore.

## Description

### Technical Field

The present invention relates to a warmer for medical treatment.

### Background Art

In general, a device is required, which warms a dehydration solution or blood at a temperature similar to a body temperature to be injected when the hydration solution or blood is injected into a human body.

The dehydration solution or blood is kept at a low temperature in order to prevent the dehydration solution or blood from being decayed or a harmful material from being generated, and when the dehydration or blood in a low-temperature state is input into the human body, energy by patient's metabolism is required to make the input dehydration solution or blood be the same as the body temperature. Moreover, when the dehydration solution or blood in the low-temperature state is input into the human body, a body temperature of a patient decreases and thus, worse, heart shock is raised to lead up to death and in particular, a general anesthesia patient of which a body temperature is not controlled feels intense cold and stimulates a cold point even in a kin into the dehydration solution or blood is inputted to allow the patient to fell cold pain.

Therefore, before a fluid such as the dehydration solution or blood is input into a body of the patient, the dehydration solution or blood needs to be warmed at a temperature similar the body temperature.

Further, when the dehydration solution or blood is warmed, air may be generated and when the generated air is not appropriately removed, the blood is prevented from being smoothly circulated and in some cases, a blood vessel or a capillary is blocked by the air to cause a cell to be necrotized, and as a result, a side effect such as embolism may occur.

### Disclosure

### Technical Problem

An embodiment of the present invention provides a warmer for medical treatment.

An embodiment of the present invention provides a warmer for medical treatment that can effectively warm a dehydration solution or blood.

An embodiment of the present invention provides a warmer for medical treatment that prevents a partition of the warmer from being damaged by heat at the time of warming a dehydration solution or blood.

An embodiment of the present invention provides a warmer for medical treatment that can reduce air generated from a dehydration solution or blood by heat at the time of warming a dehydration solution or blood.

An embodiment of the present invention provides a warmer for medical treatment that can effectively remove and discharge air in a dehydration solution or blood.

An embodiment of the present invention provides a warmer for medical treatment in which a dehydration solution or blood can be smoothly moved.

### Technical Solution

A warmer according to an embodiment includes: a heater inlcuding a resist pattern for generating heat on front and back surfaces of the heater; a partition and cover respectively disposed on the front and back surfaces of the heater to surround the heater, thereby forming a passage, through which a fluid flows, together with the heater; a first connection part for introducing the fluid into the passage; a second connection part for discharging the fluid from the passage; a pore formed in the cover; a filter disposed on an inner surface of a portion of the cover in which the pore is formed; and an air discharger coupled to the cover to discharge air contained in the fluid through the filter and the pore.

### Effect of Invention

An embodiment of the present invention provides a warmer for medical treatment.

An embodiment of the present invention provides a warmer for medical treatment that can effectively warm a dehydration solution or blood.

An embodiment of the present invention provides a warmer for medical treatment that prevents a partition of the warmer from being damaged by heat at the time of warming a dehydration solution or blood.

An embodiment of the present invention provides a warmer for medical treatment that can reduce air generated from a dehydration solution or blood by heat at the time of warming a dehydration solution or blood.

An embodiment of the present invention provides a warmer for medical treatment that can effectively remove and discharge air in a dehydration solution or blood.

An embodiment of the present invention provides a warmer for medical treatment in which a dehydration solution or blood can be smoothly moved.

### Description of Drawings

FIG. 1 is a perspective view of a warmer for medical treatment according to an embodiment of the present invention.

FIG. 2 is a diagram illustrating a state in which the warmer for medical treatment according to the embodiment of the present invention is assembled.

FIG. 3 is a front view of a heater.

FIG. 4 is a bottom view of the heater.

FIG. 5 is a diagram illustrating a state in which a first cover and a heater are coupled to each other in the warmer for medical treatment according to the embodiment of the present invention is assembled.

FIG. 6 is a diagram illustrating a state in which the first cover, the heater, and a second cover are coupled to each other in the warmer for medical treatment according to the embodiment of the present invention is assembled.

FIG. 7 is a diagram describing a state in which a connector in a lower case, and a power applied electrode and a sensing electrode of the heater according to the warmer for medical treatment according to the embodiment of the present invention.

FIG. 8 is a diagram illustrating a state in which the first cover, the heater, the second cover, and a third cover are coupled to each other in the warmer for medical treatment according to the embodiment of the present invention is assembled.

FIGS. 9 to 12 are diagrams describing a warmer according to another embodiment.

FIG. 13 is a diagram describing a warmer according to another embodiment of the present invention.

FIG. 14 is a diagram describing a warmer according to yet another embodiment of the present invention.

FIGS. 15 and 16 are diagrams an electrical connection relationship of a power connector.

FIG. 17 is a diagram describing a layout of a resist pattern and a second partition.

FIG. 18 is a diagram describing a warmer according to another embodiment of the present invention.

FIG. 19 is a diagram illustrating yet another example of the heater.

FIGS. 20 to 22 are diagrams describing a warmer according to yet another embodiment of the present invention.

FIGS. 23 to 24 are diagrams describing a warmer according to yet another embodiment of the present invention.

FIG. 25 is a diagram describing a warmer according to yet another embodiment of the present invention.

FIG. 26 is a diagram describing a warmer according to yet another embodiment of the present invention.

FIG. 27 is a diagram describing a warmer according to yet another embodiment of the present invention.

FIG. 28 is a diagram describing a warmer according to yet another embodiment of the present invention.

FIG. 29 is a diagram describing a warmer according to yet another embodiment of the present invention.

### Best Mode

In describing an embodiment of the present invention, when each layer (film), area, pattern or structure are referred to as being formed "on" or "under", "on" and "under" include both "directly" and "indirectly via another layer". Further, a reference for "on" or "under" of each layer will be described based on the drawings.

In the drawings, the thickness or size of each layer is enlarged or omitted, or schematically illustrated for easy and clear description. Further, an actual size is not fully reflected to the size of each component.

Hereinafter, a warmer for medical treatment according to an embodiment of the present invention will be described in detail with reference to the accompanying drawings.

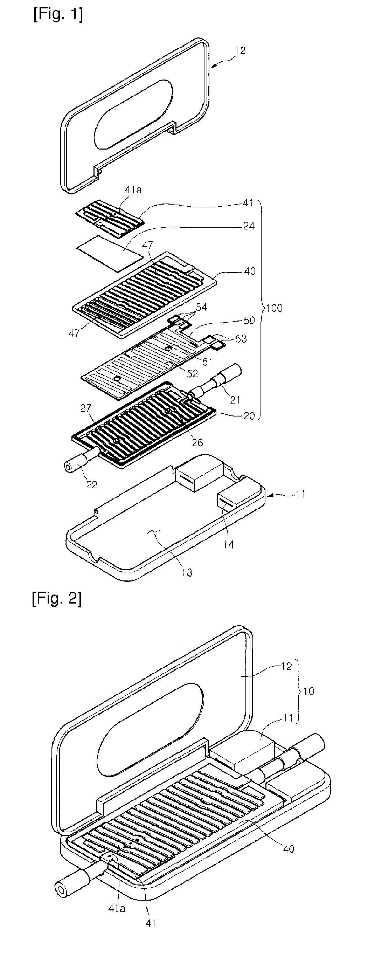

FIG. 1 is a perspective view of a warmer for medical treatment according to an embodiment of the present invention. FIG. 2 is a diagram illustrating a state in which the warmer for medical treatment according to the embodiment of the present invention is assembled.

Referring to FIGS. 1 and 2, the warmer for medical treatment according to the embodiment of the present invention may include a case 10 configured by coupling a lower case 11 and an upper case 12. A warmer 100 is placed on a warmer mounting part 13 between the lower case 11 and the upper case 12.

The warmer 100 according to the embodiment of the present invention a warming function to warm a fluid such as a dehydration solution or blood and an air filter function to remove air contained in the fluid.

Further, the warmer 100 according to the embodiment of the present invention may prevent a partition of the warmer 100 from being damaged by heat at the time of warming the dehydration solution or blood.

In order to provide the functions, the warmer 100 according to the embodiment may include a first cover 20, a heater 50, a second cover 40, a filter 24, and a third cover 41.

The first cover 20 and the second cover 40 is coupled to each other with the heater 50 interposed therebetween and the second cover 40 and the third cover 41 may be coupled to each other with the filter 24 interposed therebetween.

The second cover 40 and the third cover 41 are formed as separate members in the embodiment, but the second cover 40 and the third cover 41 need not particularly be separated from each other in terms of a characteristic of preventing the partition of the warmer 100 from being damaged by heat and the second cover 30 and the third cover 41 may be formed as one body like the first cover 20. That is, the second cover 40 is formed in a similar shape as the first cover 20 and the third cover 41 may not be formed.

In this case, the filter 24 is not placed between the third cover 41 and the second cover 40 and may be formed in the second connection part 22.

A plurality of first partitions 27 are formed on a surface of the first cover 20 facing the heater 50 and a plurality of second partitions 47 are formed on a surface of the second cover 40 facing the heater 50. The first cover 20 and the first partition 27 may be integrally injected and similarly, the second cover 40 and the second partition 47 may be integrally injected.

The second partition 47 is formed in the second cover 40 to form a passage to correspond to the first partition 27 of the first cover 20 and only the second partition 47 is formed in a partial area of the second cover 40 and a part of the second cover 40 supporting the second partition 47 in an upper direction is not formed. The second partition is supported on the second cover 40 at both ends thereof.

The filter 24 is placed and the third cover 41 is for forming the passage while supporting the filter 24 is placed, in a part where the second cover 40 is partially removed. A plurality of pores 41a for discharging air generated from respective passages partitioned by the second partition 47 are formed in the second cover 41.

The first partition 27 is inclined in a first direction and the second partition 47 is inclined in a second direction different from the first direction to form the passage together with the heater 50.

A passage formed by the first cover 20 including the first partition 27, the second cover 40 including the second partition 47, the third cover 41, and the heater 50 is formed to cover the heater 50 in a thread form to sufficiently transfer heat generated from the heater 50 to a fluid that flows through the passage.

Since the width of the heater 50 is smaller than a width of the first partition 27 and the second partition 47, a front passage of the heater 50 and a rear passage of the heater partitioned by the heater 50 are connected to each other on both surfaces of the heater 50. That is, the front passage and the rear passage are connected to each other to cover the heater 50.

The first cover 20 includes the first connection part 21 into which the fluid is introduced and the second connection part 22 from which the fluid is discharged. Therefore, the first cover 20 including the partition 27, the heater 50, the second cover 40 including the second cover 47, and the third cover 41 are coupled to each other, the fluid is introduced from the first connection part 21, passes through the passage formed by the first cover 20 including the first partition 27, the second cover 40 including the second partition 47, the third cover 41, and the heater 50, and is discharged through the second connection part 22.

Since the warmer 100 is used while the first connection part 21 is placed in an upper side and the second connection part 22 is placed in a lower side, the fluid introduced through the first connection part 21 is discharged through the second connection part 22 along the passage.

For example, the fluid introduced through the first connection part 21 flows in the first direction in which the first partition 27 is inclined through a rear passage formed by a rear surface of the heater 50, the first cover 20, and the first partition 27 of the first cover 20 and flows in the second direction in which the second partition 47 of the second cover 40 is inclined from the end of the rear passage through a front passage formed by a front surface of the heater 50, the second cover 40, and the second partition 47 of the second cover 40.

That is, when a direction in which the first connection part 21 is placed is set as an upper direction and a direction in which the second connection part 22 is placed is set as a lower direction, the fluid introduced through the first connection part 21 flows in an upper right direction in which the first partition 27 is inclined through a rear passage formed by the rear surface of the heater 50, the first cover 20, and the first partition 27 of the first cover 20 and flows in a lower left direction in which the second partition 47 is inclined through a front passage formed by the front surface of the heater 50, the second cover 40, and the second partition 47 of the second cover 40 from the end of the rear passage.

In addition, the fluid flows to the second connection part 22 through a rear passage formed by the rear surface of the heater 50, the first cover 20, and the first partition 27 of the first cover 20 and a front passage formed by the front surface of the heater 50, the second partition 47 of the second cover 40, and the third cove 41 from the end of the rear passage, in a part adjacent to the second connection part 22.

As illustrated in the figure, the filter 24 is placed further adjacent to the second connection part 22. Since more air is generated from the fluid in a bubble form in a warmed state rather than a low-temperature state, it is more effective in providing the air filter function that the filter 24 is placed adjacent to the second connection part 22.

As such, a passage formed by the first cover 20 including the first partition 27, the heater 50, the second cover 40 including the second partition 47, and the third cover 41 is formed to cover the heater 50 in the thread form, and as a result, the fluid introduced through the first connection part 21 is sufficiently warmed by the heater 50.

Since the fluid is introduced through the first connection part 21 by predetermined pressure, the pressure of the fluid that flows through the passage is higher than external pressure of the second cover 40 and the third cover 41 and in this case, the air contained in the fluid is discharged to the outside through the filter 24. Therefore, the air is removed from the fluid and the fluid is discharged through the second connection part 22.

The filter 24 is placed above the front passage to be placed horizontally to the flow of the fluid. For example, a membrane having micro-cavities as a member having hydrophobic which is small in affinity with the fluid may be used for the air filter 24. For example, the hydrophobic membrane may be used for the air filter 24 and in detail, an EmflonPTFE product made by Pall Corporation may be adopted. The micro-cavities have such a size that the air may be discharged but the fluid does not pass therethrough. The filter 24 may be attached to the third cover 41 by using a laser, a low-frequency wave, a high-frequency wave, an ultrasonic wave, and an adhesive.

A plurality of pores 41a are formed in the third cover 41 to allow the air collected by the filter 24 to be discharged to the outside.

Although not illustrated, the plurality of pores 41a, the filter 24, and the third cover 41 that provide the air filter function may be formed on the rear passage as well as the front passage.

Since the filter 24 is placed at a location facing the heater 50, the warmer 100 according to the embodiment may perform both the warming function and the air removing function.

In the warmer 100 according to the embodiment, the first connection part 21, the first cover 20, and the second connection part 22 may be integrally inj ected. Further, in the warmer 100 according to the embodiment, the second cover 40 and the second partition 47 may be integrally injected.

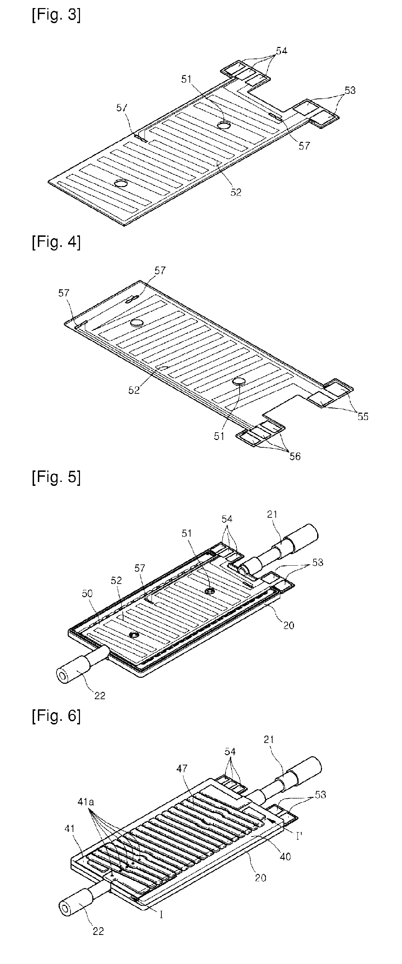

The heater 50 may be a printed circuit board (PCB) with a resist pattern 52. FIG. 3 is a front view of a heater. FIG. 4 is a bottom view of the heater.

Referring to both FIGS. 3 and 4, the resist pattern 52 is formed on the front surface and the rear surface of the heater 50, and as a result, heat is emitted from the resist pattern 52 by power applied through power applied electrodes 53 and 55. Further, a temperature sensor 57 is formed on the front surface and the rear surface of the heater 50 to sense the temperature of the fluid and send a sensed signal to the outside through sensing electrodes 54 and 56.

In the warmer 100 according to the embodiment, at least a part of the resist pattern 52 is placed between the first partitions 27 or between the second partitions 47. At least a part may be inclined in an inclination direction of the first partitions 27 or the second partitions 47. That is, at least a part of the resist pattern 52 is spaced from the first partitions 27 or the second partitions 47 to extend in parallel. The resist pattern 52 is partially formed below the first and second partitions 27 and 47 in order to connect the resist patterns 52 partitioned by the first and second partitions 27 and 47, and is formed in only a minimum dimension below the first and second partitions 27 and 47.

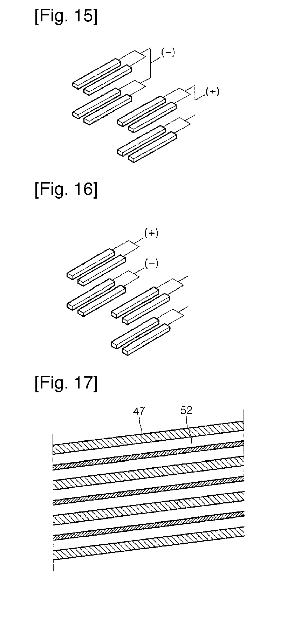

As illustrated in FIG. 17, the resist pattern 52 is placed between the second partitions 47 and extends to be spaced from the second partitions 47 by a predetermined gap. Of course, as illustrated in FIGS. 3 and 4, in order to connect the resist pattern 52 to two power applied electrodes 53, a part of the resist pattern 52 may be placed below the second partitions 47 and in the embodiment of the present invention, a dimension where the second partitions 47 and the resist pattern 52 cross each other, that is, a dimension of the resist pattern 52 placed below the second partitions 47 is configured to be minimum.

As illustrated in FIG. 17, when the resist pattern 52 extends in parallel to the second partitions 47, it is advantageous that the heat generated from the resist pattern 52 may be effectively transferred to the fluid that flows along the passage and in particular, the heat generated from the resist pattern 52 is transferred to the second partitions 47 to reduce a problem that the second partitions 47 are damaged by the heat.

The second partitions 47 and the resist pattern 52 are illustrated in FIG. 17, but may be similarly applied even between the first partitions 27 and the resist pattern 52.

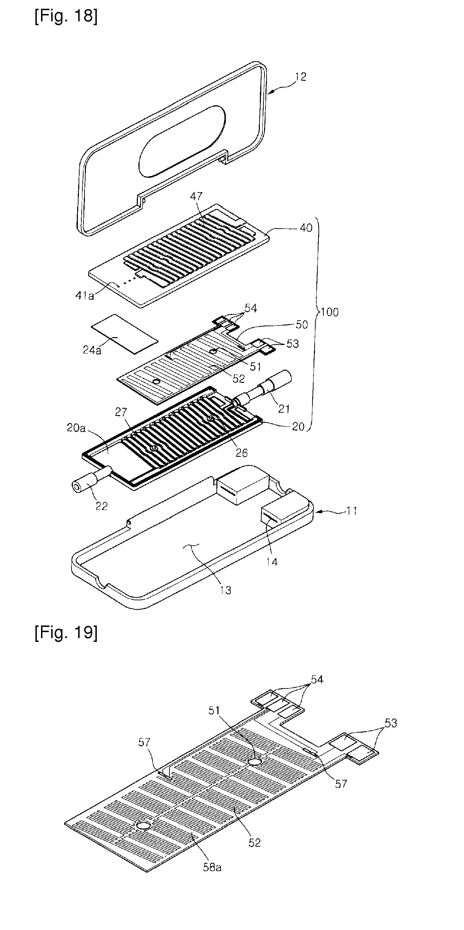

FIG. 19 illustrates yet another example of the heater.

As illustrated in FIG. 19, the resist pattern 52 is minimally less formed in a part 58a contacting the second partitions 47, and the resist pattern 52 that extends from any one of the power applied electrodes 53 and the resist pattern 52 that extends from the other one are place at one side and the other side based on a virtual straight line that crosses the center of the heater 50 in a length direction, respectively. In addition, the resist pattern 53 may be connected with a short side of the heater 50 where the power applied electrode 53 is placed in a part adjacent to the short side, which is placed in an opposite direction.

A resist pattern density of a portion of the resist pattern 53 contacting the second partitions 47 is lower than that of a portion placed between the second partitions 47.

In an example illustrated in FIG. 19m the resist pattern 52 is formed by a plurality of lines in a region between the second partitions 47 unlike the resist pattern 52 illustrated in FIG. 17.

Meanwhile, as described below, the resist patterns 52 formed on the front surface and the rear surface of the heater 50 may be connected to each other in series or in parallel while supplying power through a power connector 14a.

In the warmer 100 according to the embodiment, the resist pattern 52 is formed on the front surface and the rear surface of the heater 50 in order to effectively perform warming, since the heater 50 is manufactured by omitting a process of electrically connecting the resist pattern 52 of the front surface and the resist pattern 52 of the rear surface to each other, the resist patterns 52 formed on the front surface and the rear surface are electrically separated from each other.

That is, powers of + and - are applied to two power applied electrodes 53 formed on the front surface of the heater 50, respectively and similarly, powers of + and - are applied to two power applied electrodes 53 formed on the rear surface of the heater 50, respectively.

For example, when the resist patterns 52 formed on the front surface and the rear surface of the heater 50 are electrically separated from each other, the process of electrically connecting the resist pattern 52 placed on the front surface of the heater 50 and the resist pattern 52 placed on the rear surface of the heater 50 is required and to this end, since a via-hole buried with a conductive material is formed in the heater 50 or a separate additional work is required, such as welding or riveting, when the resist pattern 52 of the front surface and the resist pattern 52 of the rear surface are electrically connected to each other, the conductive material is attached to even the resist pattern 52 of the front surface and the resist pattern 52 of the rear surface while the conductive material is buried in the via-hole, and as a result, it is difficult to produce the resist pattern 52 having an accurate resistance value.

Further, since the thickness of the conductive material buried in the via-hole may be smaller than that of the resist pattern 52, more heat is generated from the conductive material in the via-hole to cause a short-circuit when power is applied, and additional cost is generated during forming the via-hole to cause production cost of the heater 50 to increase.

Therefore, the resist pattern 52 placed on the front surface of the heater 59 and the resist pattern 52 placed on the rear surface are configured to be electrically separated from each other and the respective resist patterns 52 are manufactured to have the power applied electrodes 53 and 55 connected with the powers of + and -.

Similarly, the temperature sensors 57 are also formed on the front surface and the rear surface of the heater 50, respectively to be electrically separated from each other and are electrically connected with the sensing electrodes 54 and 56 formed on the front surface and the rear surface of the heater 50, respectively.

Further, a hole 51 may be formed in the heater 50 and a projection 26 may be formed in the first cover 20. The projection 26 is inserted into the hole 51 to allow the heater 50 to be placed at an accurate location on the first cover 20.

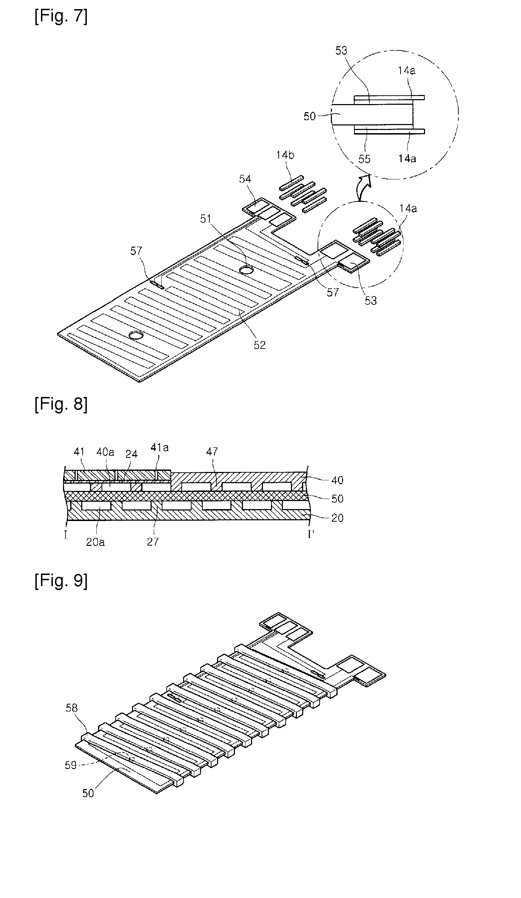

FIG. 5 is a diagram illustrating a state in which a first cover and a heater are coupled to each other in the warmer for medical treatment according to the embodiment of the present invention is assembled. FIG. 6 is a diagram illustrating a state in which the first cover, the heater, and a second cover are coupled to each other in the warmer for medical treatment according to the embodiment of the present invention is assembled. FIG. 7 is a diagram describing a state in which a connector in a lower case, and a power applied electrode and a sensing electrode of the heater according to the warmer for medical treatment according to the embodiment of the present invention.

Referring to FIGS. 5 to 7, the heater 50 is inserted between the second cover 40 and the first cover 20, and the power applied electrode 53 and 55 and the sensing electrode 54 and 56 of the heater 50 are projected to the outside.

The power applied electrode 53 and 55 and the sensing electrode 54 and 56 of the heater 50 that are projected to the outside are inserted into an electrode insertion groove 14 of the lower case 11 illustrated in FIG. 1 to be electrically connected with the power connector 14a and a sensor connector 14b placed in the lower case 11 as illustrated in FIG. 2.

The power connector 14a and the sensor connector 14b placed in the electrode insertion groove 14 are illustrated in FIG. 7. Since the power connector 14a and the sensor connector 14b need to perform a function to electrically connect the resist pattern 52 and the temperature sensor 57 formed on the front surface and the rear surface of the heater 50, which is a role of the via-hole buried with the conductive material, and a function to supply power from the outside, a dual connector structure in which connectors are placed in upper and lower parts of the heater 50 is suitable.

The connectors are formed at locations corresponding to the power applied electrodes 53 and 55 and the sensing electrodes 54 and 56 formed on the front surface and the rear surface of the heater 50, respectively.

Therefore, when the power applied electrodes 53 and 55 and the sensing electrodes 54 and 56 are inserted into the electrode insertion groove 14, the power applied electrodes 53 and 55 and the sensing electrodes 54 and 56 formed on the front surface and the rear surface of the heater 50, respectively are electrically connected with both the power and sensor connectors 14a and 14b.

Although not illustrated, a driving chip for driving the heater 50, a sensing signal processing unit for processing a signal acquired from the temperature sensor 57, a display unit for a user, an input button for a user operation, and the like, which are electrically connected with the power and sensor connectors 14a and 14b may be formed in the lower case 11.

Meanwhile, FIGS. 15 and 16 are diagrams describing an electrical connection relationship of a power connector.

The resist patterns 52 formed on the front surface and the rear surface of the heater 50 are connected in parallel in FIG. 15 and the resist patterns 52 formed on the front surface and the rear surface of the heater 50 are connected in series in FIG. 16.

Since the resist pattern 52 formed on the surface of the heater 50 and the resist pattern 52 formed on the rear surface of the heater 50 are electrically separated from each other, the resist patterns 52 formed on the front surface and the rear surface of the heater 50 may be electrically connected to each other by using the power connector 14a.

Therefore, the power is applied to the power applied electrode 53 and 55 formed on the front surface and the rear surface of the heater 50, which may be electrically connected to each other. Of course, since resistance of the resist pattern 52 depends on a serial connection method or a parallel connection method, when a desired heat emission amount in the heater 50, that is, desired resistance is set, a connection method of the resist patterns 50 of the front surface and the rear surface and resistances of the resist patterns 52 formed on the front surface and the rear surface, respectively are together considered.

FIG. 8 is a diagram illustrating a state in which the first cover, the heater, the second cover, and a third cover are coupled to each other in the warmer for medical treatment according to the embodiment of the present invention is assembled.

Referring to FIG. 8, the first cover 20, the first partition 27 of the first cover 20, and the rear surface of the heater 50 form a rear passage 20a and the second cover 40, the second partition 47 of the second cover 40, the third cover 41, and the front surface of the heater 50 form a front passage 40a.

The rear passage 20a and the front passage 40a cross each other with the heater 50 interposed therebetween at the center of the warmer 100 in the directions of the first partition 27 and the second partition 47.

The diagram illustrated in FIG. 8 is a cross-sectional view at a location inclined to one side at the center of the warmer 100. Therefore, the first partition 27 and the second partition 47 are placed to cross each other.

Meanwhile, when the fluid passes through the front passage 40a on which the filter 24 is formed, the air contained in the fluid is collected by the filter 24 to be discharged to the outside through the pore 41 a.

FIGS. 9 to 12 are diagrams describing a warmer according to another embodiment.

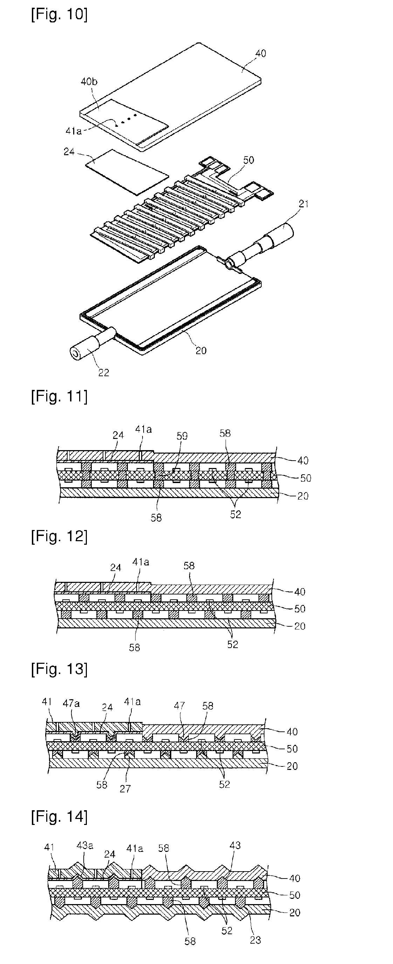

FIG. 9 illustrates a state in which a partition 58 surrounds the heater 50 in a thread form, FIG. 10 illustrates an exploded perspective view of the first cover 20, the heater 50, the filter 24, and the second cover 40, FIG. 11 illustrates a cross section at the center of the warmer in the state in which the first cover 20, the heater 50, the filter 24, and the second cover 40 are coupled, and FIG. 12 illustrates a cross section at a location inclined to one side at the center of the warmer in the state in which the first cover 20, the heater 50, the filter 24, and the second cover 40 are coupled.

Referring to FIGS. 9 to 12, in an embodiment described in FIGS. 9 to 12, the partition 58 is directly formed in the heater 50 like the aforementioned embodiment. The partition 58 is insert-injected together with the heater 50 to be attached to the heater 50. As described above even in the embodiment, a dimension where the partition 58 and the resist pattern 52 cross each other, that is, a dimension of the resist pattern 52 placed below the partition 58 is configured to be minimum.

A coating material (not illustrated) may be formed on the front surface and the rear surface of the heater 50, and when the partition 58 is insert-injected, the coating material is melted to prevent a micro crack from being generated between the partition 58 and the heater 50. In this case, as the coating material, a material which has a lower melting point than a material forming the partition 58 may be selected.

In the embodiment, connection holes 50 may be formed at the center of the heater 50 with a regular interval. The connection hole 59 allows the material forming the partition 58 to be injected into the connection hole 59 when the partition 58 is insert-injected into the heater 50 to more rigidly couple the partitions 58 formed on the front surface and the rear surface of the heater 50 to the heater 50.

The partition 58 is insert-injected together with the heater 50 and attached to the heater 50 and the partition is not formed in the first cover 20 and the second cover 40.

Meanwhile, a filter attachment part 40b is formed in the second cover 40, and as a result, the filter 24 may be attached to the second cover 40. The filter attachment part 40b may be formed by making the thickness of the second cover 40 be smaller than those of other parts or formed to be proj ected upward.

In addition, the pore 41a is formed at a location of the filter attachment part 40b corresponding to the passage to discharge the air collected by the filter 24 to the outside, similarly as in FIG. 8.

FIG. 13 is a diagram describing a warmer according to another embodiment of the present invention.

Referring to FIG. 13, the partitions 58 are formed on the front surface and the rear surface of the heater 50 as illustrated in FIG. 9 and the first partition 27 and the second partition 47 are formed even in the first cover 20 and the second cover 40, respectively.

Further, a third cover 41 having a third partition 47a may be further formed, and the third cover 41 may be formed integrally with the second cover 40 as illustrated in FIG. 11.

The partition 58 formed in the heater 50 is coupled to the heater 50 through insert-injection, and the first partition 27, the second partition 47, and the third partition 47a are injected simultaneously when the first cover 20, the second cover 40, and the third cover 41 are injected.

An end of the partition 58 is concave and ends of the first partition 27, the second partition 47, and the third partition 47a are convex. Therefore, the first partition 27, the second partition 47, and the third partition 47a may correspond to the partition 58, and as a result, the fluid may smoothly move through a predetermined passage.

Meanwhile, the filter 24 is formed between the heater 50 including the partition 58 and the third cover 41 including the third partition 47a, and the pore 41a is formed in the third cover 41 to perform the function to remove the air contained in the fluid as described above.

Meanwhile, the end of the partition 58 is concave, and the ends of the first partition 27, the second partition 47, and the third partition 47a are convex in FIG. 13, but end of the partition 58 may be convex and the ends of the first partition 27, the second partition 47, and the third partition 47a may be concave.

FIG. 14 is a diagram describing a warmer according to yet another embodiment of the present invention.

Referring to FIG. 14, the partitions 58 are formed on the front surface and the rear surface of the heater 50 as illustrated in FIG. 9 and grooves 23 and 43 are formed at locations of the first cover 20 and the second cover 40 corresponding to the partition 58.

Further, a third cover 41 having a third partition 43a may be further formed, and the third cover 41 may be formed integrally with the second cover 40 as illustrated in FIG. 11.

The partition 58 formed in the heater 50 is coupled to the heater 50 through insert-injection, and grooves 20a, 40a, and 43a of the first cover 20, the second cover 40, and the third cover 41 are formed when the first cover 20, the second cover 40, and the third cover 41 are injected.

The end of the partition 58 is convex and a part of the partition 58 is inserted into the grooves 20a, 40a, and 43a of the first cover 20, the second cover 40, and the third cover 41. Therefore, the grooves 20a, 40a, and 43a of the first cover 20, the second cover 40, and the third cover 41 may correspond to the partition 58, and as a result, the fluid may smoothly move through a predetermined passage

Meanwhile, the filter 24 is formed between the heater 50 including the partition 58 and the third cover 41 including the groove 43a, and the pore 41a is formed in the third cover 41 to perform the function to remove the air contained in the fluid as described above.

FIG. 18 is a diagram describing a warmer according to another embodiment of the present invention.

The warmer 100 illustrated in FIG. 18 has a similar shape as the warmer illustrated in FIG. 1. However, as illustrated in FIG. 18, the lengths of the first cover 20 an the second cover 40 are larger than the length of the heater 50 and thus, the heater 50 is placed at a part of a region where the first cover 20 and the second cover 40 face each other, and as a result, the first partition 27 and the second partition 47 are formed in the first cover 20 and the second cover 40, respectively.
In addition, a first space part 20a and a second space part without the first partition 27 and the second partition 47 are formed. Although the second space part is not illustrated, the second space part is formed in the second cover 40 to face the first space part 20a.

In a space formed by the first space part 20a and the second space part 40a, the air contained in the fluid that passes through the passage is discharged through the plurality of pores 41a. That is, the region where the first cover 20 and the second cover 40 face each other may be divided into a warming region where the heater 50 is placed and a filter region where the plurality of pores 41 a are placed, and the filter region is placed adjacent to the second connection part 22. A filter 24a is placed below the plurality of pores 41a, that is, in the second space part to be attached to the second cover 40.

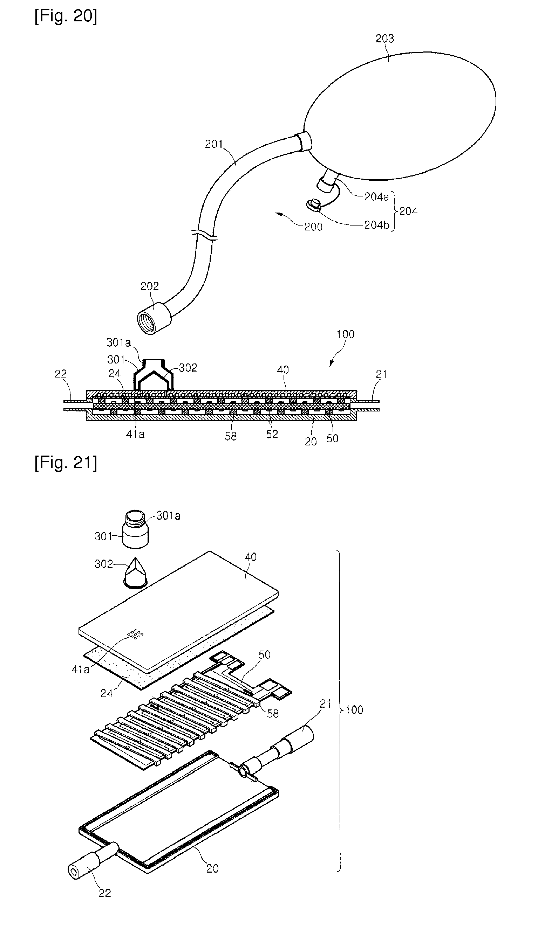

FIGS. 20 to 22 are diagrams describing a warmer according to yet another embodiment of the present invention.

Referring to FIGS. 20 to 22, in a warmer 100, a first cover 20 and a second cover 40 are coupled with each other, and a heater 50 is placed between the first cover 20 and the second cover 40 to form a passage.

A first connection part 21 and a second connection part 22 is formed in the first cover 20. For example, an external fluid flows into the passage through the first connection part 21, and the fluid passing through the passage may be discharged through the second connection part 22. In the embodiment, the first cover 20 and the second cover 40 are exemplified to be coupled with each other to form one cover, but the first cover 20 and the second cover 40 may be integrally formed.

Further, in the embodiment, the first and second connection parts 21 and 22 are exemplified to be coupled with the first cover 20, but the first and second connection parts 21 and 22 may be coupled with the second cover 40.

A partition 58 is formed in the heater 50, and the partition 58 forms the passage together with the first cover 20 and the second cover 40. The partition 58 may have the same structure as described in FIG. 9, and the structure of the partition 58 may be modified similarly to the embodiments described above.

At least one pore 41a is formed in the second cover 40, and a filter 24 is formed below the pore 41a. In the embodiment, nine pores 41 a are exemplified to be formed, but the number of pores 41 a and layout positions may be variously designed.

The filter 24 is placed on a surface of the first cover 20 which faces the second cover 40 to be placed horizontally to the flow of a fluid. That is, the filter 24 may be placed between the second cover 40 and the heater 50. In the case where the filter 24 is placed vertically to the flow of the fluid, the fluid such as blood dose not pass through the filter 24, and as a result, the fluid hardly moves. Accordingly, the filter 24 is placed in not the vertical but the horizontal direction to the flow of the fluid and thus the fluid may smoothly move. Since the filter 24 is described above, the duplicated description will be omitted.

since the fluid may not move by passing through the filter 24 and only air included in the fluid may pass through the filter 24, the air included in the fluid passes through the filter 24 via the pore 41a to be discharged outside. Only when pressure of the fluid is higher than external air pressure, the air included in the fluid may be discharged outside through the filter 24.

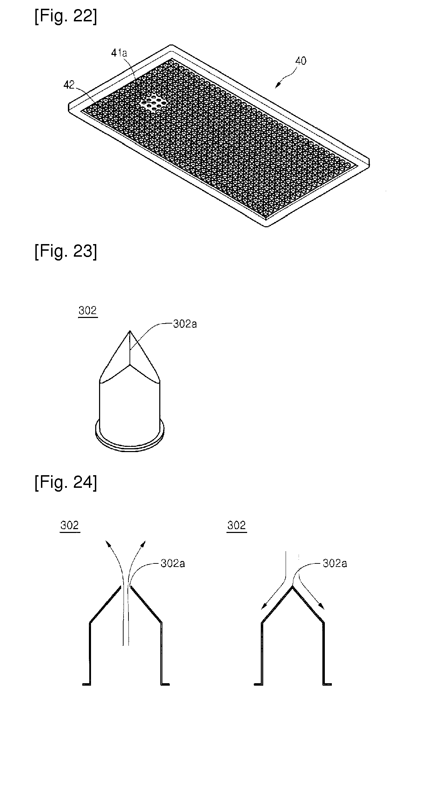

Meanwhile, a plurality of air induced projections 42 is formed on a surface of the second cover 40 facing the first cover 20. The air induced projections 42 are placed so that the filter 24 and the second cover 40 are partially spaced apart from each other, and as a result, the air passing through the filter 24 may move in a direction where the pore 41 a is placed through a space between the air induced proj ections 42. In FIG. 22, a plurality of air induced projections 42 is exemplified to be placed in a lattice form, but the form and the number of air induced projections 42 may be variously modified.

Accordingly, even though the pore 41a is formed in a part of the second cover 40, the air passing through the filter 24 may smoothly move in the direction where the pore 41 a is placed. The filter 24 may be formed to have a similar area to the second cover 40, and air may pass through most of area of the filter 24.

In the embodiment, a check valve 302 may be further formed outside of the first cover 40 with the pore 41a. The check valve 302 prevents the air discharged outside through the pore 41a from being introduced into the fluid through the pore 41a again. The check valve 302 need not particularly be formed, and may be additionally provided in order to further improve an air removing function.

A coupling unit 301 may be further formed outside of the check valve 302. The coupling unit 301 may serve to protect the check valve 302. Further, the coupling unit 301 is coupled with the check valve 302 to support the check valve 302. Further, the coupling unit 301 may serve to be coupled with an air discharger 200.

Referring to FIGS. 23 and 24, the check valve 302 may be made of a synthetic resin material having elasticity, and has minute openings 302a. For example, the check valve 302 may be made of a silicon material.

As illustrated in FIG. 23, when the air introduced from the pore 41a is discharged to the outside of the check valve 302, the openings 302a open and the check valve 302 may smoothly discharge the air. On the contrary, as illustrated in FIG. 24, when the air flows from the outside of the check valve 302 to the inside of the check valve 302, the openings 302a close and external air does not flow into the check valve 302. That is, in the check valve 302, the openings 302a open by pressure of the air when the air flows from the inside to the outside, but when the air flows from the outside to the inside, the openings 302a further contact each other by the air pressure, and as a result, the movement of the air is blocked.

Accordingly, the check valve 302 allows the air to be discharged to only the outside of the check valve 302. However, in the embodiment, only one shape of the check valve 302 is exemplified, but the shape of the check valve 302 may be variously modified.

Further, in the embodiment, the check valve 302 is exemplified to be used, but since the check valve 302 serves to effectively perform discharging of the air, the check valve 302 need not particularly be applied and may be omitted.

The warmer 100 according to the embodiment may be coupled with the air discharger 200. The air discharger 200 more effectively discharges the air in the warmer 100. The air discharger 200 forcibly lowers pressure of the outside of the cover, and as a result, the air included in the fluid may more easily pass through the filter 24. That is, the air discharger 200 forcibly sucks the air included in the fluid to the outside of the cover.

A coupling unit 302 formed in the warmer 100 may be coupled with the air discharger 200.

The air discharger 200 may include an air pocket 203 having elasticity and a substantially globular shape, a tube 201 connected with the air pocket 203, and a fastening part 202 formed at an end of the tube 201. The air pocket 203 may be made of a silicon material, and for example, the air pocket 203 may be made of a silicon gel having excellent elasticity.

Further, the air discharger 200 may further include an air discharge port 204. The air discharge port 204 may include an air discharge tube 204a, and a tube cap 204b coupled with the air discharge tube 204a.

The fastening part 202 of the air discharger 200 is coupled with the coupling unit 301. A screw groove is formed inside of the fastening part 202, and a thread 301a may be formed outside of the coupling unit 301.

When pressure is applied to the air pocket 203 in a state where the fastening part 202 and the coupling unit 301 are coupled with each other, while the air pocket 203 is compressed, the air in the air pocket 203 is discharged outside through the air discharge port 204. In addition, when the tube cap 204b is coupled with the air discharge tube 204a while pressure is applied to the air pocket 203, force to be restored to an original form by elasticity is applied to the air pocket 203. In this case, since external air may not flow through the air discharge port 204, the pressure in the air pocket 203 is lower than external pressure of the air pocket 203, and as a result, the air is more actively discharged through the pore 41 a.

Meanwhile, the air discharge port 204 need not particularly be formed. For example, before the fastening part 202 is coupled with the coupling unit 301, the air in the air pocket 203 is discharged outside by applying pressure to the air pocket 203. In this state, when the fastening part 202 is coupled with the coupling unit 301, the same effect may be obtained without forming the air discharge port 204.

However, in the case where the air discharge port 204 is formed, while the fastening part 202 is coupled with the coupling unit 301, since the air discharge port 204 is opened to discharge the air in the air pocket 203 to the outside, the air in the air pocket 203 may be discharged many times, and as a result, there is an advantage in that the air may be more effectively discharged.

Meanwhile, a vacuum pump may be installed instead of coupling the air discharger 200.

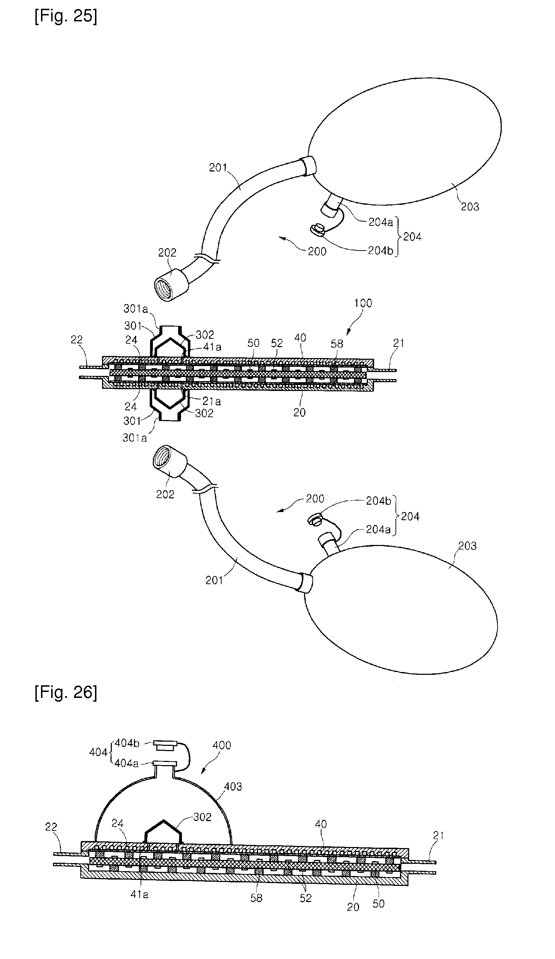

FIG. 25 is a diagram describing a warmer according to yet another embodiment of the present invention.

Referring to FIG. 25, a warmer 100 illustrated in FIG. 25 has a similar structure to the warmer 100 illustrated in FIG. 20. Accordingly, the description which is duplicated with the description for the warmer 100 illustrated in FIG. 20 will be omitted.

However, the warmer 100 illustrated in FIG. 25 may discharge air even through not only a second cover 40 but also a first cover 20. That is, at least one pore 21a is formed in the first cover 20 similarly to the second cover 40, and a filter 24 is formed at an upper side of the pore 21a. The filter 24 is placed on a surface of the first cover 20 which faces the second cover 40 to be placed horizontally to the flow of a fluid.

A check valve 302 may be formed outside of the first cover 20 with the pore 21a. The check valve 302 prevents the air discharged outside through the pore 21a from being introduced into the fluid through the pore 21a again. The check valve 302 need not particularly be formed, and may be additionally provided in order to further improve an air removing function.

A coupling unit 301 may be further formed outside of the check valve 302. The coupling unit 301 may serve to protect the check valve 302. Further, the coupling unit 301 is coupled with the check valve 302 to support the check valve 302. Further, the coupling unit 301 may serve to be coupled with an air discharger 200.

Since the warmer 100 illustrated in FIG. 25 discharges air though the first cover 20 and the second cover 40, there is an advantage in that an air discharging effect is more excellent.

FIG. 26 is a diagram describing a warmer according to yet another embodiment of the present invention.

Referring to FIG. 26, the pore 41a may be formed in the second cover 40, and the check valve 302 may be formed outside of the pore 41a.

The check valve 302 may be coupled with the second cover 40 to be supported. Since the check valve 302 is described in FIGS. 23 and 24, the duplicated description thereof will be omitted.

Further, in the embodiment, the check valve 302 is exemplified to be used, but since the check valve 302 serves to effectively perform discharging of the air, the check valve 302 need not particularly be applied and may be omitted.

An air discharger 400 in another form is coupled with the second cover 40. In the embodiment, the air discharger 400 is exemplified to be coupled with the second cover 40, but so long as the air discharger 400 is placed to surround the pore 41a, a detailed coupling position may be variously modified. Further, the air discharger 400 may be integrally formed together with the second cover 40.

The air discharger 400 may include an air pocket having a substantially hemispheric shape and elasticity, and an air discharge port 404 connected with the air pocket 403. The air discharge port 404 may include an air discharge tube 404a, and a tube cap 404b coupled with the air discharge tube 404a.

When pressure is applied to the air pocket 403 in an open state of the air discharge tube 404a, while the air pocket 403 is compressed, the air in the air pocket 403 is discharged outside through the air discharge port 404. In addition, when the tube cap 404b is coupled with the air discharge tube 404a while pressure is applied to the air pocket 403, force to be restored to an original form by elasticity is applied to the air pocket 403. In this case, since external air may not flow through the air discharge port 404, the pressure in the air pocket 403 is lower than external pressure of the air pocket 403, and as a result, the air is more actively discharged through the pore 41a.

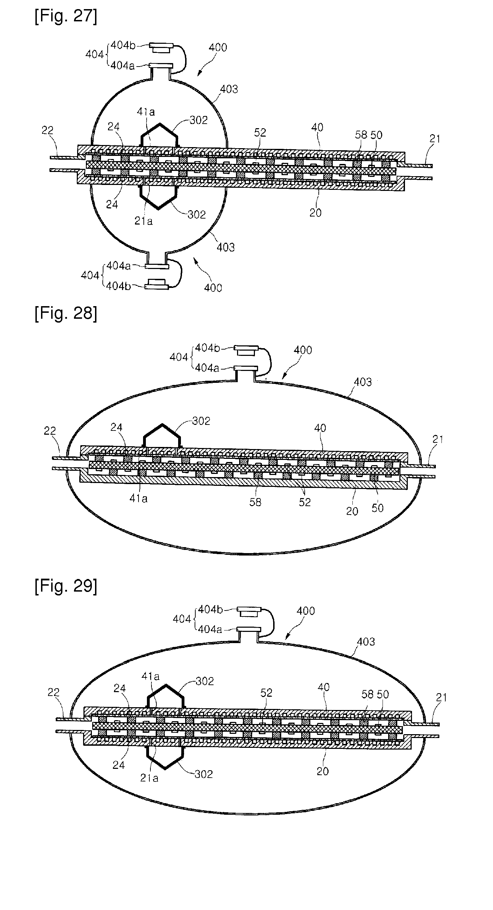

FIG. 27 is a diagram describing a warmer according to yet another embodiment of the present invention.

Referring to FIG. 27, a warmer illustrated in FIG. 27 has a similar structure to the warmer illustrated in FIG. 26. Accordingly, the description duplicated with the description for the warmer illustrated in FIG. 26 will be omitted.

However, the warmer illustrated in FIG. 27 may discharge air though the first cover 20 as well as the second cover 40. That is, at least one pore 21a is formed in the first cover 20 similarly to the second cover 40, and a filter 24 is formed at an upper side of the pore 21a. The filter 24 is placed on a surface of the first cover 20 which faces the second cover 40 horizontally to the flow of a fluid.

A check valve 302 may be formed outside of the first cover 20 with the pore 21a. The check valve 302 prevents the air discharged outside through the pore 21a from being introduced into the fluid through the pore 21a again. The check valve 302 need not particularly be formed, and may be additionally provided in order to further improve an air removing function.

The air discharger 400 described in FIG. 26 is coupled with the second cover 20. In the embodiment, the air discharger 400 is coupled with the first cover 20 as well as the second cover 40.

Since the warmer illustrated in FIG. 27 discharges air though the first cover 20 and the second cover 40, there is an advantage in that an air discharging effect is more excellent.

FIG. 28 is a diagram describing a warmer according to yet another embodiment of the present invention. In description for a warmer illustrated in FIG. 28, the description duplicated with the contents described in FIG. 26 will be omitted.

Referring to FIG. 28, an air discharger 400 having another form is coupled with an outside of a cover including the first cover 20 and the second cover 40.

The air discharger 400 may include an air pocket having a circular or oval shape, and an air discharge port 404 connected with the air pocket 403.

The first connection part 21 and the second connection part 22 are connected to the outside by passing through one side and the other side of the air pocket 403, and the first cover 20 and the second cover 40 are placed in the air pocket 403.

Unlike the warmer illustrated in FIG. 26, in the embodiment illustrated in FIG. 28, the first cover 20, the second cover 40, and the check valve 302 are included in the air pocket 403, except for the first connection part 21 and the second connection part 22 through which the fluid flows and is discharged.

Therefore, the air pocket 403 illustrated in FIG. 28 contains more air than the air pocket 403 illustrated in FIG. 26. In addition, when the air discharge tube 404a is closed by the tube cap 404b of the air discharge port 404 after the air is discharged through the air discharge port 404 by applying pressure to the air pocket 403, while the air pocket 403 is restored to the original form by elasticity, the air included in the fluid may be removed by larger force.

FIG. 29 is a diagram describing a warmer according to yet another embodiment of the present invention.

Referring to FIG. 29, a warmer illustrated in FIG. 29 has a similar structure to the warmer 100 illustrated in FIG. 28. Accordingly, the description duplicated with the description for the warmer illustrated in FIG. 28 will be described.

However, the warmer illustrated in FIG. 29 may discharge air though the first cover 20 as well as the second cover 40. That is, at least one pore 21a is formed in the first cover 20 similarly to the second cover 40, and a filter 24 is formed at an upper side of the pore 21a. The filter 24 is placed on a surface of the first cover 20 which faces the second cover 40 horizontally to the flow of a fluid.

A check valve 302 may be formed outside of the first cover 20 with the pore 21a. The check valve 302 prevents the air discharged outside through the pore 21a from being introduced into the fluid through the pore 21a again. The check valve 302 need not particularly be formed, and may be additionally provided in order to further improve an air removing function.

Since the warmer illustrated in FIG. 29 discharges air though the first cover 20 and the second cover 40, there is an advantage in that an air discharging effect is more excellent.

Although the embodiment has been primarily described as above, the embodiment is just an example and does not limit the present invention and it can be appreciated by those skilled in the art that various modifications and applications not described above can be made within the scope without departing from the spirit of the embodiment. For example, each component described in detail in the embodiment can be modified. In addition, it should be appreciated that differences associated with the modifications and applications are included in the scope of the present invention defined in the appended claims.

### Industrial Applicability

An embodiment of the present invention may provide a warmer for medical treatment.

An embodiment of the present invention may provide a warmer for medical treatment capable of effectively warming a dehydration solution or blood.

An embodiment of the present invention may provide a warmer for medical treatment capable of reducing a partition of the warmer from being damaged by heat when warming a dehydration solution or blood.

An embodiment of the present invention may provide a warmer for medical treatment capable of reducing air generated in a dehydration solution or blood by heat when warming the dehydration solution or the blood.

An embodiment of the present invention may provide a warmer for medical treatment capable of effectively removing and discharging air in a dehydration solution or blood.

An embodiment of the present invention may provide a warmer for medical treatment capable of smoothly moving a dehydration solution or blood.

## Claims

1. A warmer for medical treatment, comprising:
a heater including a resist pattern for generating heat on front and back surfaces of the heater;
a partition and cover respectively placed on the front and back surfaces of the heater to surround the heater, thereby forming a passage, through which a fluid flows, together with the heater;
a first connection part for introducing the fluid into the passage;
a second connection part for discharging the fluid from the passage; a pore formed in the cover;
a pore formed in the cover;
a filter placed on an inner surface of a portion of the cover in which the pore is formed; and
an air discharger coupled to the cover to discharge air contained in the fluid through the filter and the pore.

2. The warmer of claim 1, wherein:
the air discharger further includes an air pocket coupled to the cover and having elasticity and an air discharge port formed on one side of the air pocket, the air discharge port including an air discharge tube and a tube plug coupled to the air discharge tube.

3. The warmer of claim 2, wherein:
the cover, the pore, and the filter are placed in the air pocket, and
the first connection part and the connection part are exposed to one side and the other side of the air pocket through the air pocket.

4. The warmer of claim 1, wherein:
a check valve is coupled to an outer surface of a part where the pore of the cover is formed.

5. The warmer of claim 4, wherein:
a coupling unit is placed on an exterior of the check valve in the cover,
the air discharger is coupled to the coupling unit, and
the air discharger includes a fastening part coupled to the coupling unit, a tube that extends from the fastening part, and an air pocket coupled to the tube and having elasticity.

6. The warmer of claim 1, wherein:
at least a part of the resist pattern is formed in parallel to the partition.

7. The warmer of claim 1, wherein:
the resist pattern is formed between two adjacent partitions among the partitions in the form of a plurality of lines.

8. The warmer of claim 1, wherein:
the partition and cover include a first partition and a first cover placed on a rear surface of the heater, and a second partition and a second cover placed on a front surface of the heater to form the passage to corresponding to the first partition and the first cover.

9. The warmer of claim 8, wherein:
the first partition is placed in a first inclination direction and the second partition is placed in a second inclination direction different from the first inclination direction.

10. The warmer of claim 8, wherein:
a region between the first cover and the second cover includes a warming region where the heater is placed and a filter region where the heater is not placed, and
the first partition and the second partition are placed in the warming region and the pore is formed in and the filter is coupled to the filter region.

11. The warmer of claim 8, wherein:
the pore is formed in each of the first cover and the second cover, and the filter is each placed between the first cover and the partition and between the second cover and the partition.

12. The warmer of claim 1, wherein:
the filter is placed in parallel to a movement direction of the fluid.

13. The warmer of claim 1, wherein:
a plurality of air guide projections are formed on an inner surface of the cover, and
the air guide projections contact the filter to make the cover and the filter be partially separated from each other.

14. The warmer of claim 1, wherein:
the heater is formed by a PCB.

15. The warmer of claim 1, wherein:
a part of the heater is projected to the outside of the cover and power applied electrodes are formed on a front surface and a rear surface of a proj ected portion of the heater.

16. The warmer of claim 1, wherein:
a temperature sensor and a sensing electrode connected with the temperature sensor are formed on a front surface and a rear surface of the heater.

17. The warmer of claim 15, wherein:
two power applied electrodes are formed on each of the front surface and the rear surface of the heater to be connected in series or in parallel through a power connector.
